# EUROPEAN PATENT APPLICATION

(11) **EP 4 797 209 A1**
(43) Date of publication of application: **26.08.2026**
(21) Application number: 25159594.8
(22) Date of filing: 24.02.2025
(51) Int. Cl.: G06T 11/00, A61B 6/03

(54) **MODIFIED RAW PROJECTION DATA IN COMPUTED TOMOGRAPHY**

(71) Applicant: Koninklijke Philips N.V., 5656 AE Eindhoven (NL)
(72) Inventor: KOEHLER, Thomas, Eindhoven (NL); GRASS, Michael, Eindhoven (NL)
(74) Representative: Philips Intellectual Property & Standards

(57) **Abstract**

Disclosed herein is a method of computed tomography that comprises receiving measured raw projection data (122) of a computed tomography scan descriptive of a field of view (320) of a subject (308). The method further comprises reconstructing (200) a measured computed tomography image (124) from the raw projection data and receiving (202) a modified computed tomography image (126). At least a central region (800) of the modified computed tomography image is identical with the measured computed tomography image. The method further comprises generating (204) a difference image (128) by subtracting the measured computed tomography image from the modified computed tomography image and generating (206) synthetic raw projection data (132) descriptive of the difference image. The method further comprises generating (208) modified raw projection data (134) by adding the synthetic raw projection data to the measured raw projection data. The method further comprises providing (210) the modified raw projection data.

## Description

### TECHNICAL FIELD

The invention relates to computed tomography, in particular to the generation of modified raw projection data.

### BACKGROUND

In Computed Tomography (CT), X-ray measurements are taken from a variety of angles around a subject and used to reconstruct cross-sectional or tomographic images. A CT scanner or system may have an X-ray source and detector that rotate around the subject to acquire the X-ray measurement at the variety of angles. The data acquired by the detectors is referred to as the raw projection data.

United States patent application publication US2020118317A1 discloses a computer-implemented method for obscuring one or more facial features of a subject in an image. A head of the subject is detected in the image and a location of one or more facial features of the subject is identified in the image. A region of the image to modify is determined based on the location of the one or more facial features. The determined region comprises a part of the head on which the one or more facial features are located. The image within the determined region is modified to obscure the one or more facial features.

### SUMMARY

The invention provides for a method of computed tomography, a computer program product, and a medical system in the independent claims. Embodiments are given in the dependent claims.

In one aspect a method of computed tomography is disclosed. The method comprises receiving measured raw projection data of a computed tomography scan descriptive of a field of view of a subject. The method further comprises reconstructing a measured computed tomography image from the raw projection data. The method further comprises receiving a modified computed tomography image. At least a central region of the modified computed tomography image is identical with the measured computed tomography image. The method further comprises generating a difference image by subtracting the measured computed tomography image from the modified computed tomography image. The method further comprises generating synthetic raw projection data descriptive of the difference image. The method further comprises generating modified raw projection data by adding the synthetic raw projection data to the measured raw projection data. The method further comprises providing the modified raw projection data.

In another aspect, a computer program product is disclosed. The computer program product comprises a computer readable storage medium having machine executable instructions embodied therewith. The machine executable instructions are configured to implement an example of the method.

In another aspect, a medical system is disclosed. The medical system comprises a memory storing machine executable instructions. The medical system further comprises a computational system. Execution of the machine executable instructions causes the computational system to receive measured raw projection data of a computed tomography scan descriptive of a field of view of a subject. Execution of the machine executable instructions further causes the computational system to reconstruct a measured computed tomography image from the raw projection data. Execution of the machine executable instructions further causes the computational system to receive a modified computed tomography image. At least a central region of the modified computed tomography image is identical with the measured computed tomography image. Execution of the machine executable instructions further causes the computational system to generate a difference image by subtracting the measured computed tomography image from the modified computed tomography image. Execution of the machine executable instructions further causes the computational system to generate synthetic raw projection data descriptive of the difference image. Execution of the machine executable instructions further causes the computational system to generate modified raw projection data by adding the synthetic raw projection data to the measured raw projection data. Execution of the machine executable instructions further causes the computational system to provide the modified raw projection data.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates a cloud-based example of a medical system.
Fig. 6 shows several different images which are used to illustrate examples of the method.
Fig. 7 shows several additional images which are further used to illustrate examples of the method.
Fig. 8 shows an additional set of images which may be used to explain examples of the method.

### DESCRIPTION OF EMBODIMENTS

Like numbered elements in these Figures are either equivalent elements or perform the same function. Elements which have been discussed previously will not necessarily be discussed in later Figures if the function is equivalent.

In one example, there is a method of computed tomography. The method comprises receiving the measured raw projection data of a computed tomography scan descriptive of a field of view of the subject. The raw projection data is the X-ray intensity measurements collected by a CT system or scanner before any image reconstruction is performed. Potentially the raw projection data represents the amount of X-ray attenuation along different paths through the scanned object. The measured raw projection data refers to a particular set of raw projection data that was acquired using a computed tomography system. The method further comprises reconstructing a measured computed tomography image from the raw projection data.

The method further comprises receiving a modified computed tomography image. At least a central region of the modified computed tomography image is identical with the measured computed tomography image. The central region may for example be a central region within the subject as depicted in the field of view. It is therefore possible that there is a difference between the measured computed tomography image and the modified computed tomography image in a boundary or edge region.

The method further comprises generating a difference image by subtracting the measured computed tomography image from the modified computed tomography image. This subtraction may for example be performed voxel-wise. There may be a voxel in the measured computed tomography image which corresponds to a particular voxel in the modified computed tomography image. The difference image is performed by subtracting these voxels from the measured computed tomography image from the corresponding voxels of the modified computed tomography image. This difference image shows or illustrates the difference between the two computed tomography images.

The method further comprises generating synthetic raw projection data descriptive of the difference image. For example, modeling may be used to generate the synthetic raw projection data from the difference image. The method further comprises generating modified raw projection data by adding the synthetic raw projection data to the measured raw projection data. For example, there may be lines or measurements in the measured raw projection data that correspond to the modified raw projection data. For example, the synthetic raw projection data may have been generated by using a model or simulation of the computed tomography system that was used to measure the raw projection data. In this case, then the synthetic raw projection data and the measured raw projection data would have the same format.

The modified raw projection data is then generated by adding the synthetic raw projection data to the measured raw projection data. The method further comprises providing the modified raw projection data. This example may be beneficial because it provides a means of generating raw projection data that corresponds to the modified computed tomography image. Often times, when the computed tomography scans are shared they are shared by providing the raw projection data format. If one generates a synthetic or partially synthetic or modified computed tomography image one would not be able to share the data in this format. Examples of this method, for example, may be used to provide this modified raw projection data for a modified computed tomography image which corresponds to the central region of the measured computed tomography image.

In another example, the modified raw projection data is provided within a DICOM file. A DICOM file is a Digital Imaging and Communication in Medicine file and is a standard format which is used to share medical images and medical imaging data. The DICOM file may contain the image, the raw projection data, and/or metadata descriptive of the subject. Providing the modified raw projection data within a DICOM file may enable it to be used for various automated systems such as performing for image reconstruction and image segmentation.

In another example, the DICOM file preferably comprises a standardized DICOM tag. The standardized DICOM tag as used herein may for example represent a DICOM tag which is generated independent of various specifics of a particular subject. This may for example be useful in providing data which can be used for training machine learning systems. Instead of providing, for example, patient data, the data which is relevant to a particular training routine for an artificial intelligence may be used instead. Using the standardized DICOM tag also eliminates the possibility of sharing sensitive patient data accidentally. This may for example provide better security when using the DICOM file.

In another example, the synthetic raw projection data is generated by modeling the difference image using a simulated computed tomography system. In this example, one could perform this modeling by doing several steps. One would be to define the geometry of the CT scanner, which would include the location of X-ray sensors as well as the beam path, such as a parallel beam, fan beam, or cone-beam for the CT system. The next step would be to simulate the X-ray attenuation through the image. As there is a CT image available, in this case the difference image, one could use this to simulate the X-ray attenuation directly. From this, one could then know what the data measured by the detectors defined in the geometry would be. This would then be used for providing the synthetic raw projection data.

In another example, the field of view comprises a head region of the subject. In some instances, the entire head of the subject may be within the field of view. The method comprises receiving a replacement computed tomography image. The method comprises registering a replacement computed tomography image to the measured computed tomography image. For example, one may have a replacement computed tomography image from a variety of sources. This could be an image of the same individual, it could be a computed tomography image of a different individual, it could be data that is referenced to an anatomical atlas, it could be an image of a computed tomography phantom of a head, or it may even be a synthetically generated computed tomography image such as generated by an AI. By registering it, there is then a mapping between the replacement computed tomography image and the measured computed tomography image. There is then a correspondence between the voxels in both of these computed tomography images. The method further comprises receiving a boundary region of the measured computed tomography image. This for example may be straightforward to perform. For example, an edge detection algorithm could be used for finding an edge of the subject's head within the computed tomography image. Then a distance from these detected edges could be defined as the boundary region. This would be straightforward to perform using an algorithmic method.

The method further comprises generating the modified computed tomography image by replacing the boundary region of the measured computed tomography image at least partially with the image data from the registered replacement computed tomography image. As there is a mapping between the voxels of the two, one can take a voxel within the boundary region and then look this up in the replacement computed tomography image and replace that voxel with the value from the replacement computed tomography image.

In one example one could copy the voxels from the replacement computed tomography image into this boundary region. This may result in having edges within the modified computed tomography image. Another way to perform this would be to have an averaging or linear combination of the values from the voxels in the replacement computed tomography image and the measured computed tomography image. For example, at the edge of the boundary region the voxels could be set so that they are completely taken from the replacement computed tomography image. At the part of the boundary, which is within the subject, one could then take it completely from the measured computed tomography image or just a mixture or portion of the two voxels. One could envision using a linear curve or any continuous curve to decide how the various combinations are performed. This may be advantageous because it may provide for a smooth transition within the boundary region such that the resulting modified computed tomography image looks realistic.

The method further comprises providing the modified computed tomography image. This may be beneficial because it may provide for a computed tomography image which has a replacement surface with the original CT data from the measured computed tomography image. This may be useful in a variety of situations. This may for example be useful for modifying the face such as for performing facial reconstruction or planning for surgery for facial reconstruction or plastic surgery. It may also be used for obscuring features of the subject for providing for CT images which have a standardized appearance.

In another example, the method further comprises detecting a facial region in the measured computed tomography image using a facial recognition algorithm. The method further comprises cancelling generation of the modified computed tomography image if the facial recognition is not detected. For example, an artificial intelligence, such as a classifier or an image which assigns a bounding box, may be used to identify the facial region. In the case of training a neural network, one may provide labeled images, for example, an image that has the facial region identified in a bounding box. This may, for example, be used by a deep learning algorithm to train the neural network. For example, if one is performing a facial reconstruction or needs the modified computed tomography image for some other purpose when working on the subject's face, then it may be beneficial to cancel the generation if the face is not present.

In another example, the replacement of the boundary region of the measured computed tomography image is limited to the facial region. As was mentioned before, for applications where it is advantageous to replace the facial region, it may not be necessary to replace the entire boundary region. For example, if one is performing a facial reconstruction then there may be no benefit in replacing the back portion of the subject's head. This may for example lead to more accurate or more rapid generation of the modified computed tomography image.

In another example, the replacement of the boundary region of the measured computed tomography image is performed throughout the entire boundary region. **In** this example, the entire outer surface of the measured computed tomography image is replaced with the surface of the replacement computed tomography image. This for example may be useful when one wants to standardize the appearance of the computed tomography images.

In another example, replacing the boundary region of the measured computed tomography image is performed using an interpolation between the measured computed tomography image and the registered replacement computed tomography image. As was stated before, this may be beneficial because it may provide for a means where the modified computed tomography image looks more realistic.

In another example, the interpolation is linear. The various other interpolations may be used, however, using a linear interpolation may be straightforward mathematically and may produce results quickly.

In another example, the replacement computed tomography image is descriptive of a computed tomography phantom. In this example, for example a scan of the computed tomography phantom, may be performed using a computed tomography system and this is used to provide the replacement computed tomography image. This may then be registered to the measured computed tomography image and used to then generate the modified computed tomography image. This may be particularly useful in instances where it is beneficial to standardize the appearance of the computed tomography image.

In another example, the replacement computed tomography image is a clinical computed tomography image. A clinical computed tomography image as used herein, encompasses a real computed tomography image and not one that is generated synthetically or is artificial. For example, the clinical computed tomography image could be acquired from the same or a different subject. This may be beneficial because the use of a clinical computed tomography image may be useful in generating a modified computed tomography image that looks real.

In another example, the clinical computed tomography image is of the subject. In this case, the clinical computed tomography image was acquired from the same subject as the measured computed tomography image. This may be useful in several situations, for example, if the subject has damage to the facial region or has a facial surgery planned, the modified computed tomography image may be useful for planning or performing one of these activities.

In another example, the method further comprises providing a prior computed tomography image of the subject and then generating the clinical computed tomography image by modifying the prior computed tomography image using simulated facial implants. The simulated facial implants may for example be collagen or bone which is used to modify the outer structure or surface of the subject's face. This may for example be useful because the clinical computed tomography image is essentially a modification or mashup of the actual image of the subject. This may for example be useful for planning facial surgery as well as reconstructive surgery or, in some cases, plastic surgery.

In another example, the method further comprises controlling a computed tomography system to acquire the measured raw projection data. In some cases, the simulated computed tomography system may be modeled to match the computed tomography system. In this case, then the simulated and the actually measured raw projection data are compatible.

In another example, there is a computer program product which comprises a computer readable storage medium that has machine executable instructions stored on it. The machine executable instructions are configured to implement an example of a method as described above. This may be beneficial because it may provide for a means of upgrading a computer or computational system for performing various methods.

In another aspect, there is a medical system which comprises a memory storing machine executable instructions and a computational system. Execution of the machine executable instructions causes the computational system to receive measured raw projection data of a computed tomography scan descriptive of a field of a subject. Execution of the machine executable instructions further causes the computational system to reconstruct a measured computed tomography image from the raw projection data. Execution of the machine executable instructions further causes the computational system to receive a modified computed tomography image. At least a central region of the modified computed tomography image is identical with the measured computed tomography image.

Execution of the machine executable instructions further causes the computational system to generate a difference image by subtracting the measured computed tomography image from the modified computed tomography image. Execution of the machine executable instructions further causes the computational system to generate synthetic raw projection data descriptive of the difference image. Execution of the machine executable instructions further causes the computational system to generate modified raw projection data by adding the synthetic raw projection data to the measured raw projection data. Execution of the machine executable instructions further causes the computational system to provide the modified raw projection data. The advantages of this system have been previously discussed.

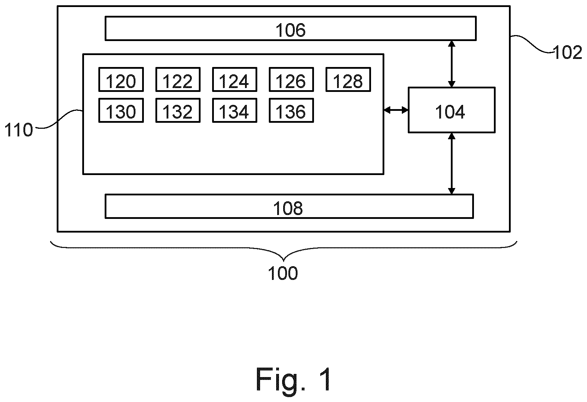

Fig. 1 illustrates an example of a medical system 100. The medical system 100 is shown as comprising a computer 102. The computer may represent one or more computer systems located at one or more locations. For example, various computational tasks described in this example may be split amongst multiple machines at multiple locations. The computer 102 is shown as comprising a computational system 104. Likewise, the computational system 104 may comprise one or more computational systems or computing cores located at one or more locations. The computational system 104 is shown as being in communication with an optional hardware or network interface 106. This hardware or network interface 106 may be used for controlling other components of the medical system 100 if they are available or for communicating with other computational systems.

The computational system 104 is shown as being in further communication with an optional user interface 108. The user interface 108 may for example be useful for such tasks as rendering or displaying images or for controlling the operation and function of the medical system 100. For example, if the medical system 100 additionally comprises a computed tomography system, the user interface 108 may contain a graphical user interface or other controls for controlling the computed tomography system.

The computational system 104 is shown as being in further communication with a memory 110. The memory 110 represents the various types of memory which are accessible to the computational system 104. In some examples the memory 110 may be a non-transitory storage medium. The memory 110 is shown as storing machine executable instructions 120. The machine executable instructions 120 may enable the computational system 104 to perform such tasks as controlling other components of the medical system 100, performing numerical tasks, and performing image processing tasks.

The memory 110 is further shown as containing measured raw projection data 122 that was acquired or received from a computed tomography system and descriptive of a field of view of a subject. The memory 110 is further shown as containing a measured computed tomography image 124 that was reconstructed from the measured raw projection data 122.

The memory 110 is further shown as storing a modified computed tomography image 126. The modified computed tomography image 126 and the measured computed tomography image 124 both comprise a central region which is identical. This means that the modified computed tomography image 126 has a peripheral or edge region which may be different than the measured computed tomography image 124. The memory 110 is further shown as containing a difference image that was constructed or generated by subtracting the measured computed tomography image 124 from the modified computed tomography image 126. This for example may be performed on a voxel-wise basis. There may be voxels in the measured computed tomography image 124 that correspond directly to the voxels of the modified computed tomography image 126.

The memory 110 may further store an optional model of a computed tomography system 130. This may be a mathematical model, which may be used to simulate the acquisition of raw projection data by the computed tomography system that acquired the measured raw projection data 122. By inputting the difference image 128 into the computed tomography system 130 synthetic raw projection data 132, which is also seen as being stored in the memory 110, may be generated. The memory 110 is further shown as containing modified raw projection data 134 that was generated or calculated by adding the synthetic raw projection data 132 to the measured raw projection data 122. The memory 136 is also shown as containing an optional DICOM file which is used to store or present the modified raw projection data 134. In some examples, the DICOM file 136 may comprise a standardized DICOM tag which may provide generic or non-personalized metadata for the DICOM file 136.

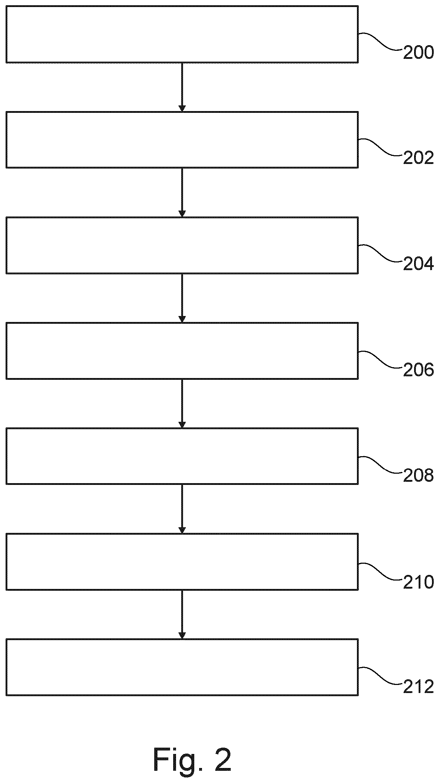

Fig. 2 shows a flowchart which illustrates a method of using the medical system 100 of Fig. 1. In step 200 the measured raw projection data 122 is received. The measured raw projection data is of a computed tomography scan descriptive of a field of view of a subject. In step 202, the measured computed tomography image 124 is reconstructed from the measured raw projection data 122. In step 204, a modified computed tomography image 126 is received. At least a central region of the modified computed tomography image 126 matches or is identical with the measured computed tomography image 124.

In step 206, a difference image 128 is generated or calculated by subtracting the measured computed tomography image 124 from the modified computed tomography image 126. In step 208, synthetic raw projection data 132 is generated that is descriptive of the difference image 128. As was mentioned, this may be done optionally by using the model of the computed tomography system 130 and the difference image 128. In step 210, the modified raw projection data 134 is generated by adding the synthetic raw projection data 132 to the measured raw projection data 122. In step 212, the modified raw projection data 134 is provided, for example it may be added to a DICOM file 136 and then sent to a workstation or provided in a database.

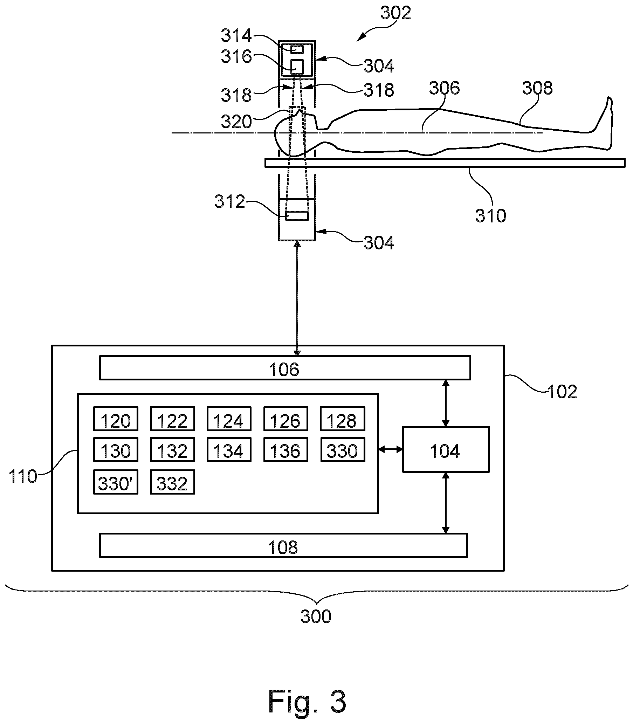

Fig. 3 illustrates a further example of a medical system 300. The medical system 300 depicted in Fig. 3 is similar to the medical system 100 in Fig. 1 except that it additionally comprises a computed tomography system 302. The computed tomography system 302 comprises a gantry 304 that rotates about an axis of rotation 306. A subject, 308, is shown as being supported by a subject support 310. Within the gantry there is a detector 312 and an X-ray source 314. There may also be a collimator 316 for collimating the X-rays 318 from the X-ray source 314. The X-rays 318 are shown as passing through a head or facial region of the subject 308. The area scanned by the X-rays 318 as it rotates around the axis of rotation 306 forms a field of view 320. In some instances, data may be taken for multiple positions of the subject 308 along the axis of rotation 306. That is to say there may be multiple slices of measured raw projection data 122 acquired. The computational system 104 controls the CT system 302 to acquire the measured raw projection data 122.

The memory 110 is further shown as containing a replacement computed tomography image 330. As it was mentioned above, this may for example be a synthetically generated computed tomography image from a neural network or other artificial intelligence system, it may be a computed tomography image of a phantom, or it may be a clinical computed tomography image that is acquired from a real person, such as the same subject 308 or a different individual. The memory 110 is also shown as containing a registered replacement computed tomography image 330'. The registered replacement computed tomography image 330' is the replacement computed tomography image 330 after it has been registered to the measured computed tomography image 124.

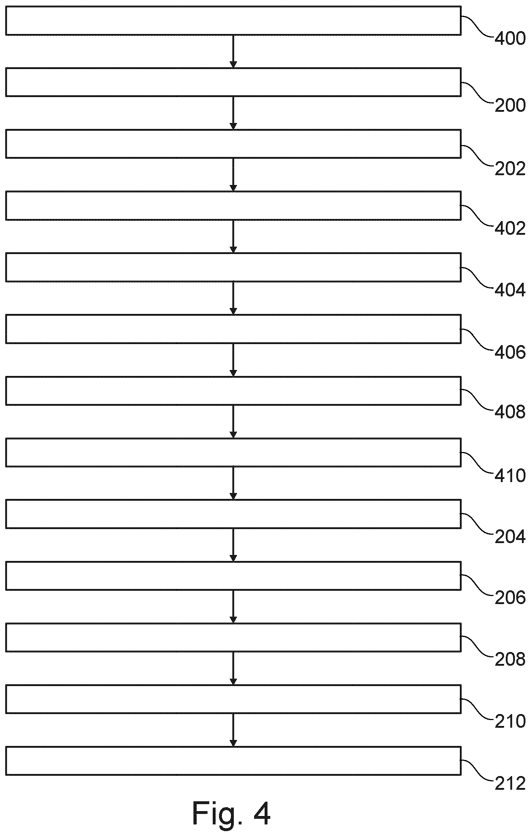

Fig. 4 shows a flowchart which illustrates a method of operating the medical system 300 of Fig. 3. In step 400 the computed tomography system 302 is controlled to acquire the measured raw projection data 122. Next, steps 200 and 202 are performed, as was illustrated in Fig. 2. In step 402, the replacement computed tomography image 330 is received. In step 404, the replacement computed tomography image 330 is registered to the measured computed tomography image 124. In step 406, a boundary region 332 is received for the measured computed tomography image 124. In step 408, the modified computed tomography image 126 is generated by replacing the boundary region of the measured computed tomography image at least partially with image data from the registered replacement computed tomography image 330'. In step 410, the modified computed tomography image is provided. Steps 204, 206, 208, 210, and 212 are performed as was illustrated in Fig. 2.

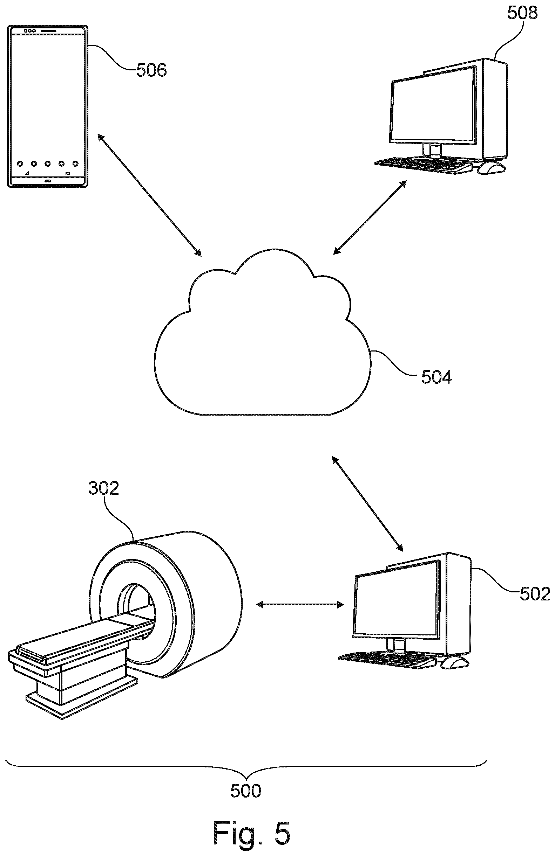

Fig. 5 illustrates an alternative medical system 500. This illustrates a cloud-based or cloud computing-based medical system 500. In this example, the computed tomography system 302 is controlled by a local controller 502. The local controller 502 communicates with a cloud-based computing system 504 that may also be in communication with a handheld telecommunication device 506, such as a smartphone, and is also in communication with a workstation 508. The various computational tasks illustrated in Figs. 1-4 may be distributed amongst the various computational devices 502, 504, 506, and 508 illustrated in Fig. 5. For example, the measured raw projection data 122 may be acquired by having the local controller 502 control the computed tomography system 302. This measured raw projection data 122 may then be transferred to the cloud-based computing system 504 where the other steps are performed. Various visualizations or DICOM files 136 may be provided to the handheld telecommunication device 506 and/or the workstation 508.

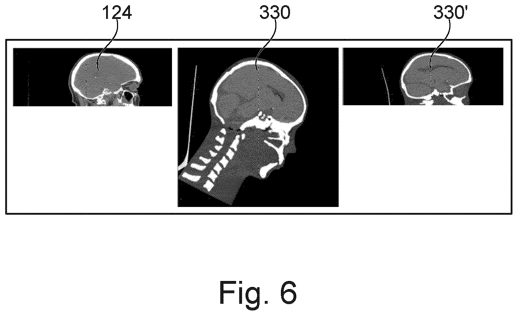

Fig. 6 shows several different images which are used to illustrate examples of the method. The image on the left is a measured computed tomography image 124. In the middle is an example of a replacement computed tomography image 330. In this particular example, the replacement computed tomography image 330 is a CT image of a head phantom. On the right is the registered replacement computed tomography image 330'. The replacement computed tomography image 330 was registered to the measured computed tomography image 124 to produce this image.

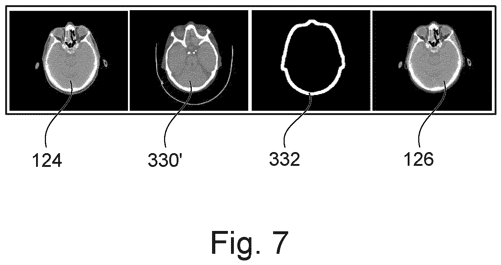

Fig. 7 shows several additional images which are further used to illustrate examples of the method. On the left is an example of a measured computed tomography image 124. The image 330' is a registered replacement computed tomography image. It is again a computed tomography image of a CT head phantom. Image 332 shows a thickened skin mask which is the boundary region 332. Voxels of the measured computed tomography image 124 are replaced or partially replaced using voxels or image data from the registered replacement computed tomography image 330'. The image 126 is the modified computed tomography image and was produced by replacing the voxels within the boundary region 332 of the measured computed tomography image 124 with image data from the registered replacement computed tomography image 330'.

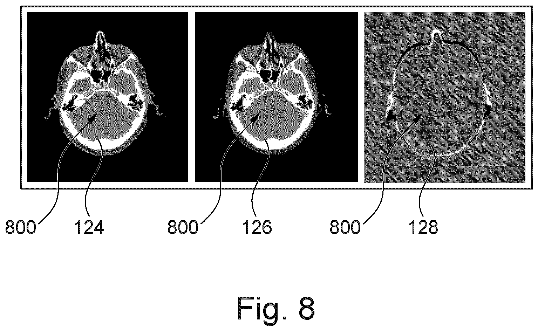

Fig. 8 shows an additional set of images which may be used to explain examples of the method. Image 124 is an example of a measured computed tomography image 124. Image 126 is a modified computed tomography image 126. Both images have a central region 800 which is identical. Image 128 is a difference image 128 that was constructed by subtracting the measured computed tomography image 124 from the modified computed tomography image 126. The difference image 128 also illustrates the large central region 800 where the measured computed tomography image 124 and the modified computed tomography image 126 are identical.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer readable storage medium" as used herein encompasses any tangible storage medium which may store instructions which are executable by a processor or computational system of a computing device. The computer readable storage medium may be referred to as a computer readable non-transitory storage medium. The computer readable storage medium may also be referred to as a tangible computer readable medium. In some embodiments, a computer readable storage medium may also be able to store data which is able to be accessed by the computational system of the computing device. Examples of computer readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer readable storage medium. Computer memory is any memory which is directly accessible to a computational system. 'Computer storage' or 'storage' is a further example of a computer readable storage medium. Computer storage is any non-volatile computer readable storage medium. **I**n some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

100 medical system
102 computer
104 computational system
106 hardware / network interface
108 user interface
110 memory
120 machine executable instructions
122 measured raw projection data
124 measured computed tomography image
126 modified computed tomography image
128 difference image
130 model of computed tomography system or simulated computed tomograph system
132 synthetic raw projection data
134 modified raw projection data
136 DICOM file
200 receiving measured raw projection data of a computed tomography scan descriptive of a field of view of a subject
202 reconstructing a measured computed tomography image from the raw projection data
204 receiving a modified computed tomography image, wherein at least a central region of the modified computed tomography image is identical with the measured computed tomography image
206 generating a difference image by subtracting the measured computed tomography image from the modified computed tomography image
208 generating synthetic raw projection data descriptive of the difference image
210 generating modified raw projection data by adding the synthetic raw projection data to the measured raw projection data
212 providing the modified raw projection data
300 medical system
302 CT system
304 gantry
306 axis of rotation
308 subject
310 subject support
312 detector
314 X-ray source
316 collimator
318 X-rays
320 field of view
330 replacement computed tomography image
330' registered replacement computed tomography image
332 boundary region (or mask)
400 controlling a computed tomography system to acquire the measured raw projection data
402 receiving a replacement computed tomography image
404 registering the replacement computed tomography image to the measured computed tomography image
406 receiving a boundary region of the measured computed tomography image
408 generating the modified computed tomography image by replacing the boundary region of the measured computed tomography image at least partially with image data from the registered replacement computed tomography image
410 providing the modified computed tomography image
500 medical system
502 local controller
504 cloud computing system
506 handheld telecommunications device
508 workstation
800 central region

## Claims

1. A method of computed tomography, wherein the method comprises:
- receiving (200) measured raw projection data (122) of a computed tomography scan descriptive of a field of view (320) of a subject (308);
- reconstructing (202) a measured computed tomography image (124) from the raw projection data;
- receiving (204) a modified computed tomography image (126), wherein at least a central region (800) of the modified computed tomography image is identical with the measured computed tomography image;
- generating (206) a difference image (128) by subtracting the measured computed tomography image from the modified computed tomography image;
- generating (208) synthetic raw projection data (132) descriptive of the difference image;
- generating (210) modified raw projection data (134) by adding the synthetic raw projection data to the measured raw projection data; and
- providing (212) the modified raw projection data.

2. The method of claim 1, wherein the modified raw projection data is provided within a DICOM file (136), wherein the DICOM file preferably comprises a standardized DICOM tag.

3. The method of claim 1 or 2, wherein the synthetic raw projection data is generated by modeling the difference image using a simulated computed tomography system (130).

4. The method claim 1, 2, or 3, wherein the field of view comprises a head region of the subject, wherein the method further comprises;
- receiving (402) a replacement computed tomography image (330);
- registering (404) the replacement computed tomography image to the measured computed tomography image;
- receiving (406) a boundary region (332) of the measured computed tomography image;
- generating (408) the modified computed tomography image by replacing the boundary region of the measured computed tomography image at least partially with image data from the registered replacement computed tomography image (330'); and
- providing (410) the modified computed tomography image.

5. The method of claim 4, wherein the method further comprises:
- detecting a facial region in the measured computed tomography image using a facial recognition algorithm; and
- cancelling generation of the modified computed tomography image if the facial recognition is not detected.

6. The method of claim 5, wherein replacement of the boundary region of the measured computed tomography image is limited to the facial region.

7. The method of claim 4, 5, or 6, wherein replacement of the boundary region of the measured computed tomography image is performed throughout the entire boundary region.

8. The method of claim 4, 5, or 6, wherein replacing the boundary region of the measured computed tomography image is performed using an interpolation between the measured computed tomography image and the registered replacement computed tomography image, and wherein the interpolation is preferably linear.

9. The method of any one of the preceding claims, wherein the replacement computed tomography image is descriptive of a computed tomography phantom.

10. The method of any one of the preceding claims, wherein the replacement computed tomography image is a clinical computed tomography image.

11. The method of claim 10, wherein the clinical computed tomography image is of the subject.

12. The method of claim 11, wherein the method further comprises:
- providing a prior computed tomography image of the subject; and
- generating the clinical computed tomography image by modifying the prior computed tomography image using simulated facial implants.

13. The method of any one of the preceding claims, wherein the method further comprises controlling (400) a computed tomography system to acquire the measured raw projection data.

14. A computer program product comprising a computer readable storage medium having machine executable instructions (120) embodied therewith, said machine executable instructions are configured to implement the method of any one of claims 1 through 13.

15. A medical system (100, 300, 500) comprising:
- a memory (110) storing machine executable instructions (120);
- a computational system (104), wherein execution of the machine executable instructions causes the computational system to:
- receive (200) measured raw projection data (122) of a computed tomography scan descriptive of a field of view (320) of a subject (308);
- reconstruct (202) a measured computed tomography image (124) from the raw projection data;
- receive (204) a modified computed tomography image (126), wherein at least a central region (800) of the modified computed tomography image is identical with the measured computed tomography image;
- generate (206) a difference image (128) by subtracting the measured computed tomography image from the modified computed tomography image;
- generate (208) synthetic raw projection data (132) descriptive of the difference image;
- generate (210) modified raw projection data (134) by adding the synthetic raw projection data to the measured raw projection data; and
- provide (212) the modified raw projection data.
